(19) Europäisches Patentamt
European Patent Office
Office européen des brevets

(11) Veröffentlichungsnummer: **0 135 778**
**B1**

(12) # EUROPÄISCHE PATENTSCHRIFT

(45) Veröffentlichungstag der Patentschrift: **21.09.88**

(51) Int. Cl.⁴: **C 07 D 285/12**

(21) Anmeldenummer: **84109743.9**

(22) Anmeldetag: **16.08.84**

(54) **Verfahren zur Herstellung von 2,5-Bis-(hydrocarbyldithio)-1,3,4-thiadiazolen.**

(30) Priorität: **27.08.83 DE 3330918**

(43) Veröffentlichungstag der Anmeldung: **03.04.85 Patentblatt 85/14**

(45) Bekanntmachung des Hinweises auf die Patenterteilung: **21.09.88 Patentblatt 88/38**

(84) Benannte Vertragsstaaten: **AT BE CH DE FR GB IT LI LU NL SE**

(56) Entgegenhaltungen: **US-A-3 087 932**
**US-A-3 663 561**

**Die Akte enthält technische Angaben, die nach dem Eingang der Anmeldung eingereicht wurden und die nicht in dieser Patentschrift enthalten sind.**

(73) Patentinhaber: **RHEIN- CHEMIE RHEINAU GMBH, Postfach 81 04 09 Mülheimer Strasse 24- 28, D-6800 Mannheim 81 (DE)**

(72) Erfinder: **Hugo, Peter, Prof.Dr. c/o Technische Universität, Strasse des 17. Juni 135, D-1000 Berlin 12 (DE)**
Erfinder: **Noack, Rainer, Dr., Schillerstrasse 70, D-1000 Berlin 12 (DE)**

(74) Vertreter: **Jochum, Axel, c/o BAYER AG Zentralbereich Patente, Marken und Lizenzen, D-5090 Leverkusen 1, Bayerwerk (DE)**

**EP 0 135 778 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Verfahren zur Herstellung von 2,5-Bis-(hydrocarbyldithio)-1,3,4-thiadiazolen, nachfolgend BHDTD bezeichnet, durch oxidative Kupplung von 2-Hydrocarbyldithio-5-mercapto-1,3,4-thiadiazol, nachfolgend HMTD bezeichnet und Hydroxycarbylmercaptan mit Wasserstoffperoxid entsprechend der Gleichung

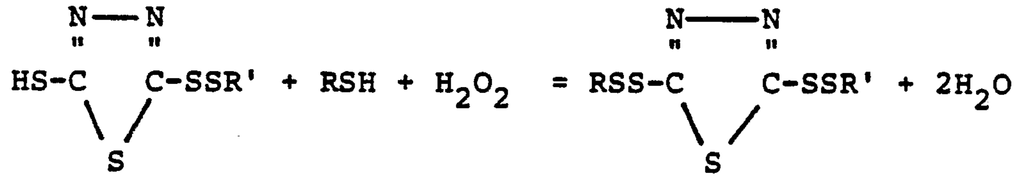

BHDTD-Verbindungen sind wirksame Korrosionsinhibitoren für die Korrosion von Buntmetallen durch aktiven Schwefel (US-PS 2 719 125 und 2 719 126).

Nach US-PS 2 719 126 sind zur Synthese Zwischenstufen mit Chlorierung erforderlich, als Nebenprodukt der Synthese entsteht Chlorwasserstoff. Auf die damit verbundenen Nachteile wird in US-PS 3 087 932 hingewiesen, wo die Herstellung von BHDTD durch Umsetzung von 2 Mol Hydrocarbylmercaptan mit 1 Mol 2,5-Dimercapto-1,3,4-thiadiazol unter Verwendung von Wasserstoffperoxid in einem Schritt zum BHDTD vorgeschlagen wird.

Der Nachteil dieser Verfahrensweise ist, daß die Umsetzung als Dreiphasenreaktion zwischen dem festen 2,5-Dimercapto-1,3,4-thiadiazol und den beiden nicht mischbaren Flüssigkeiten Mercaptan und Wasserstoffperoxidlösung in Wasser verläuft. Aus den in der zitierten Patentschrift angegebenen Produktanalysen geht hervor, daß die Umsetzung nicht vollständig verläuft und Ausbeuten von 90 % und weniger liefert.

Aufgabe des erfindungsgemäßen Verfahrens ist es, BHDTD einfach und mit einem möglichst geringen Anfall von leicht zu beseitigenden Nebenprodukten herzustellen.

Die Aufgabe wird dadurch gelöst, daß man die Verbindung der Formel

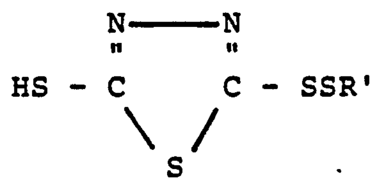

mit gleichen molaren Mengen eines Mercaptans der Formel

R - SH

worin R und R' gleich oder verschieden sind und für einen gesättigten tertiären Kohlenwasserstoffrest mit 8 bis 20 Kohlenstoffatomen stehen, in Gegenwart von Wasserstoffperoxid bei einer Temperatur zwischen 0 und 100°C umsetzt.

Vorzugsweise werden alle drei Reaktionspartner in gleichen molaren Mengen eingesetzt.

2-Hydrocarbyldithio-5-mercapto-1,3,4-thiadiazol ist bekannt. Es kann aus 2.5-Dimercapto-1.3.4-thiadiazol mit Hydrocarbylmercaptan und $H_2O_2$ hergestellt werden (vgl. US-A 3 663 561).

Als Wasserstoffperoxid kann eine handelsübliche 35 %-ige wässrige Lösung eingesetzt werden. Als Mercaptan kann auch eine entsprechende technische Mischung eingesetzt werden.

Wird mit einem molaren Einsatzverhältnis von HMTD zu Mercaptan von 1 : 1 gearbeitet, so entsteht nach dem erfindungsgemäßen Herstellungsverfahren BHDTD nahezu quantitativ ohne unerwünschte Nebenprodukte. Wird mit einem molaren Unterschuß von Hydrocarbylmercaptan und Wasserstoffperoxid gearbeitet, so erhält das Endprodukt neben nicht umgesetztem HMTD nur BHDTD ohne weitere unerwünschte Begleitstoffe. Gemische aus HMTD und BHDTD sind gleichfalls wirksame Korrosionsinhibitoren für aktiven Schwefel.

Ein weiterer Vorteil des erfindungsgemäßen Verfahrens ist, daß gezielt BHDTD-Verbindungen mit zwei verschiedenen Hydrocarbylgruppen hergestellt werden können.

Die Zusammensetzung der vom Wasser befreiten Reaktionsprodukte der nachfolgenden Beispiele wird mit einer hochdruck-flüssigkeitschromatographischen Analyse bestimmt. Bei Verwendung einer CN-Säule von 25 cm Länge der Fa. Waters und einem Eluens aus 99 Vol.-% n-Heptan und 1 Vol.-% Octanol werden bei einem Durchfluß von 2 ml/min. das eingesetzte Mercaptan, HMTD und BHDTD voneinander getrennt. Eventuell gebildete Nebenprodukte sind ebenfalls nachweisbar.

Zur quantitativen Auswertung werden die zuvor mit den reinen Substanzen geeichten Signale eines UV-Detektors ausgewertet. Die Identifizierung der Substanzen erfolgt über die Retentionszeiten.

**Beispiel 1**

35 g (0,1 mol) 2-tert.Dodecyldithio-5-mercapto-1,3,4-thiadiazol werden mit 20 g (0,1 mol) tert.Dodecylmercaptan zu einer homogenen Flüssigkeit vermischt. Zu dieser Flüssigkeit werden bei 60°C unter intensivem Rühren nach entsprechend geregelter Kühlung innerhalb von 15 Minuten 9,7 g (0,1 mol) einer 35 %-igen Wasserstoffperoxidlösung zudosiert. Das Gemisch wird unter Rühren auf 80°C aufgeheizt und bei dieser Temperatur 1 Stunde gehalten. Dann wird das Reaktionswasser bei ca. 80°C durch Vakuumverdampfen entfernt. Das Produkt besteht aus 54 g einer hellgelben, bei Raumtemperatur zähflüssigen Substanz. Nach der chromatographischen Analyse enthält sie 95 mol-% 2,5-Bis-(tert.-dodecyldithio)-1,3,4-thiadiazol.

**Beispiel 2**

35 g (0,1 mol) 2-tert.Dodecyldithio-5-mercapto-1,3,4-thiadiazol werden mit 15 g (0,075 mol) tert.Dodecylmercaptan zu einer homogenen Flüssigkeit vermischt. Zu dieser Flüssigkeit werden bei 60°C unter intensivem Rühren nach entsprechend geregelter Kühlung innerhalb von 10 Minuten 7,3 g (0,075 mol) einer 35 %-igen Wasserstoffperoxidlösung zudosiert. Die weitere Behandlung und Aufarbeitung ist die gleiche wie in Beispiel 1. Das Produkt besteht aus 49 g einer hellgelben, bei Raumtemperatur zähflüssigen Substanz. Nach der chromatographischen Analyse enthält sie 72 mol-% 2,5-Bis(dodecyldithio)-1,3,4-thiadiazol und 25 mol-% 2-tert.Dodecyldithio-5-mercapto-1,3,4-thiadiazol.

**Beispiel 3**

35 g (0,1 mol) 2-tert.Dodecyldithio-5-mercapto-1,3,4-thiadiazol werden mit 14,6 g (0,1 mol) n-Octylmercaptan zu einer homogen Flüssigkeit vermischt und mit 9,7 g (0,1 mol) einer 35 %-igen Wasserstoffperoxidlösung unter den gleichen Bedingungen wie bei Beispiel 1 umgesetzt und aufgearbeitet. Das Produkt besteht aus 49 g einer hellgelben, bei Raumtemperatur zähflüssigen Substanz. Nach der chromatographischen Analyse enthält sie 95 mol-% 2-tert.Dodecyldithio-5-n-octyldithio-1,3,4-thiadiazol.

**Patentanspruch**

Verfahren zur Herstellung von Verbindungen der Formel

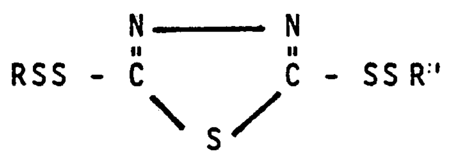

worin R und R' gleich oder verschieden sind und einen gesättigten tertiären Kohlenwasserstoffrest mit 8 bis 20 C-Atomen bedeuten, dadurch gekennzeichnet, daß man Verbindungen der Formel

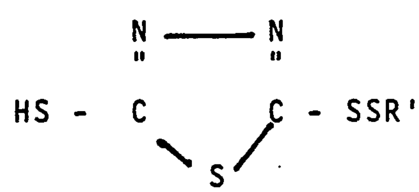

mit gleichen molaren Mengen Mercaptanen der Formel

R - SH

worin R und R' die vorstehend genannte Bedeutung haben, in Gegenwart von Wasserstoffperoxid bei einer Temperatur zwischen 0 und 100°C umsetzt.

## Claim

Process for the preparation of compounds corresponding to the following formula

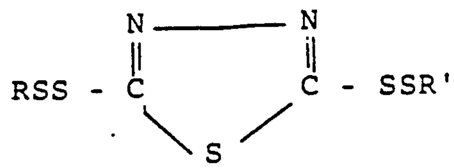

wherein R and R' may be identical or different and denote a saturated tertiary hydrocarbon group having 8 to 20 carbon atoms, characterised in that compounds corresponding to the following formula

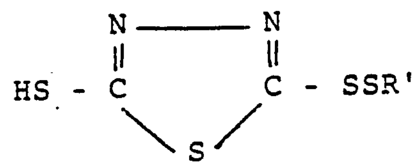

are reacted with equal molar quantities of mercaptans of the formula

R - SH

wherein R and R' have the meanings indicated above in the presence of hydrogen peroxide at a temperature from 0 to 100°C.

## Revendication

Procedé de fabrication de composés de formule

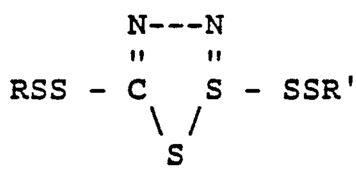

R et R' étant identiques ou différents et représentant un reste d'hydrocarbure tertiaire saturé avec 8 à 20 atomes de carbone, caractérisé en ce que l'on fait réagir des composés de formule

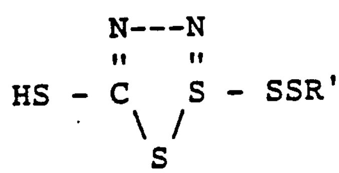

avec les mêmes quantités molaires de mercaptans présentant la formule

R - SH

R et R' ayant la signification décrite ci-dessus, en présence de peroxyde d'hydrogène, à une température située entre 0 et 100°C.